# EUROPÄISCHE PATENTSCHRIFT

(11) **EP 0 775 076 B1**
(45) Veröffentlichungstag und Bekanntmachung des Hinweises auf die Patenterteilung: **28.06.2000**
(21) Anmeldenummer: 95929846.4
(22) Anmeldetag: 11.08.1995
(51) Int. Cl.: B65D 51/16

(54) **BEHÄLTER MIT VERSCHLUSSKAPPE UND VERFAHREN ZUR GASBLASENFREIEN FÜLLUNG VON BEHÄLTERN**
CLOSURE CAP AND PROCESS FOR FILLING CONTAINERS WITHOUT FORMING GAS BUBBLES
RECIPIENT A CAPUCHON DE FERMETURE ET PROCEDE DE REMPLISSAGE DE RECIPIENTS SANS FORMATION DE BULLES DE GAZ

(30) Priorität: 11.08.1994 DE 4428434
(43) Veröffentlichungstag der Anmeldung: 28.05.1997
(73) Patentinhaber: Boehringer Ingelheim Pharma KG, 55216 Ingelheim am Rhein (DE)
(72) Erfinder: KLADDERS, Heinrich, D-45468 Mülheim (DE); FREUND, Bernhard, D-55435 Gau-Algesheim (DE); BACHTLER, Wulf, D-55126 Mainz (DE); JAEGER, Joachim, D-76646 Bruchsal (DE); EICHER, Joachim, D-44227 Dortmund (DE)
(74) Vertreter: Laudien, Dieter, Dr.
(86) Internationale Anmeldenummer: EP9503183
(87) Internationale Veröffentlichungsnummer: WO9606011

(56) Entgegenhaltungen:
- EP-A- 0 217 425
- EP-A- 0 532 873
- FR-A- 780 143
- FR-A- 1 159 909
- US-A- 1 694 851
- US-A- 2 424 801
- US-A- 2 669 370
- US-A- 3 193 993
- US-A- 4 187 893
- US-A- 5 084 042

## Beschreibung

Die Erfindung betrifft einen Behälter mit Verschlußkappe zum gasblasenfreien Füllen von Flüssigkeiten. Die Erfindung betrifft insbesondere eine Verschlußkappe, mit deren Hilfe Behälter gasblasenfrei mit Flüssigkeiten gefüllt werden können.

*Zum Verschließen von Behältern sind zwar zahlreiche Verschußmöglichkeiten bekannt. Darunter sind auch solche, die während des Verschließens ein partielles Entweichen von Luft aus dem Behälter erlauben. Einen solchen Verschluß offenbart die US -A-2 669 370. Der dort beschriebene Gegenstand betrifft einen Gummistopfen in Form eines Eintauchstutzens mit einem wulstähnlichen oberen Ende. Die dort beschriebene Konstruktion erlaubt es nicht, einen Behälter gasblasenfrei zu verschließen.*

Für manche Anwendungszwecke ist es notwendig, Flüssigkeiten so in Behälter abzufüllen, daß keine Luft- oder Gasreste mit eingeschlossen werden. Eine entsprechende Aufgabe stellt sich beispielsweise bei den Behältern für Arzneimittellösungen, welche in bestimmten Inhalationsgeräten eingesetzt werden, die Flüssigkeiten mittels einer Meßkammer dosieren (vgl. z.B. WO 91/14468, etwa Fig. 1). Da in diesem Fall die zu dosierende Flüssigkeit häufig hochwirksame Arzneistoffe enthält, die genau dosiert werden müssen, muß eine möglichst geringe Schwankungsbreite der jeweils abgegebenen Dosen gewährleistet sein; sonst erhält der Patient nicht die vom Arzt vorgesehene Wirkstoffmenge. Bei der geringen Größe des Flüssigkeitsvolumens pro Anwendung führen bei den genannten Inhalationsgeräten schon verhältnismäßig kleine Blasen zu hohen prozentualen Abweichungen.

*Die erfindungsgemäße Aufgabe wird durch die Kennzeichen der Ansprüche 1 und 10 gelöst, vorteilhafte Weiterbildungen der Erfindung sind durch die Unteransprüche gekennzeichnet.*

Die Erfindung betrifft nun einen Behälter mit Verschlußkappe und insbesondere eine Verschlußkappe und ein Verfahren (Anspruch 14) zur gasblasenfreien Füllung von Behältern. Die Verschlußkappe ist so gestaltet, daß sie beim Aufsetzen auf den Hals des Behälters einen Teil des Behälterinhalts verdrängt, gleichzeitig das Entweichen eingeschlossenen Gases ermöglicht und in der Verschlußposition einrastet. Der Verschluß kann eine Einrichtung zur Führung einer Kanüle zur Flüssigkeitsentnahme aus dem Behälter enthalten.

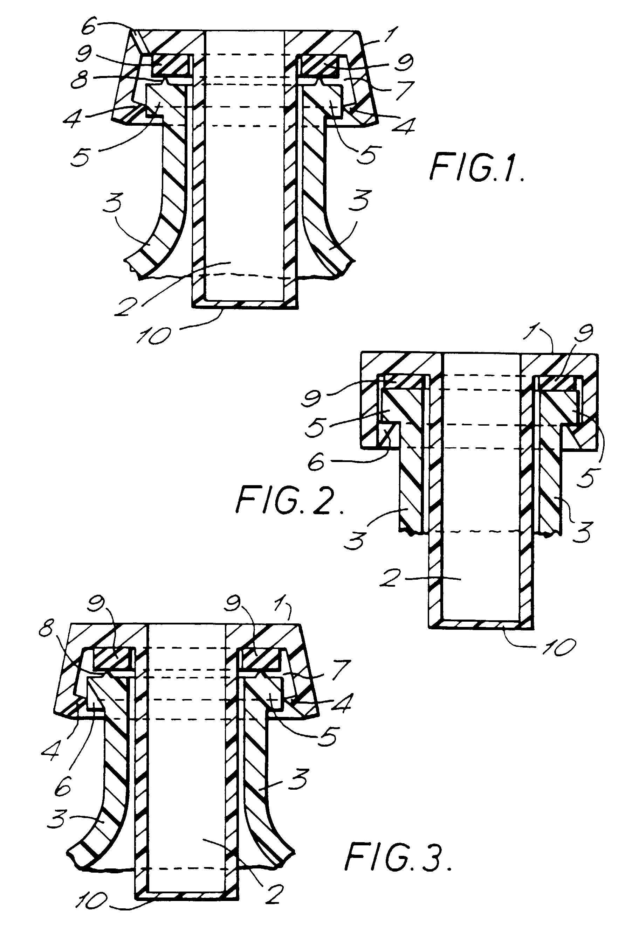

Ein axialer Schnitt durch die Mitte einer Verschlußkappe dieser Art ist in Fig. 1 dargestellt.

Fig. 2 zeigt, ebenfalls in einem axialen Schnitt, einen Behälterhals mit aufgesetzter erfindungsgemäßer Verschlußkappe in der Verschlußposition, wobei die Entlüftungsöffnungen anders angebracht sind.

Fig. 3 zeigt die erfindungsgemäße Ausführungsform, bei der die Entlüftungsöffnungen (mindestens eine) in Form von Aussparungen am oberen Teil des Behälters angeordnet sind.

Fig. 3a zeigt eine vergrößerte Ansicht der erfindungsgemäßen Ausführungsform.

Fig. 4 zeigt den erfindungsgemäßen Behälter mit aufgesetzter Verschlußkappe.

Fig. 5 zeigt eine erfindungsgemäße Verschlußkappe mit Führung für die Kanüle zur Entnahme der Flüssigkeit.

In Fig. 1 weist die elastische Verschlußkappe (1) eine Einrichtung (2) - hier in Form eines eintauchenden Stutzens - auf, durch die während des Verschließvorganges ein Teil des Inhalts aus dem Behälter (3) verdrängt wird. Ein innenliegender umlaufender Wulst (4) (Crimpkante) am unteren Rand der Verschlußkappe (1) rastet in der Verschlußposition unterhalb eines an der Außenseite des Behälterhalses umlaufenden zylindrischen Ringes (5) ein. Während die Verschlußkappe (1) aufgeschoben wird, wird der Rand der Verschlußkappe gedehnt und der Wulst (4) liegt abdichtend auf dem Ring (5) auf, so daß nur durch eine oder mehrere Entlüftungsöffnungen (6) eine Verbindung vom Kappeninnenraum (7) nach außen besteht. In der Verschlußposition wird der Zwischenraum zwischen dem flachen Teil der Verschlußkappe (1) und dem oberen Rand des Behälterhalses, der ggf. zur besseren Abdichtung mit einer umlaufenden Rippe (8) versehen ist, durch einen Dichtungsring (9) ausgefüllt und damit der Innenraum des Behälters (3) zuverlässig gegenüber dem Kappeninnenraum (7), der den Dichtungsring (9) und den Hals des Behälters (3) umgibt, abgedichtet. Der Innendurchmesser des Dichtungsrings (9) wird zweckmäßig so gewählt, daß er an der Vorrichtung (2) eng anliegt. Die Entlüftungsöffnung(en) (6) kann (können) sich auch an anderen Stellen der Kappenaußenseite befinden, etwa seitlich in dem zylindrischen Teil der Kappe.

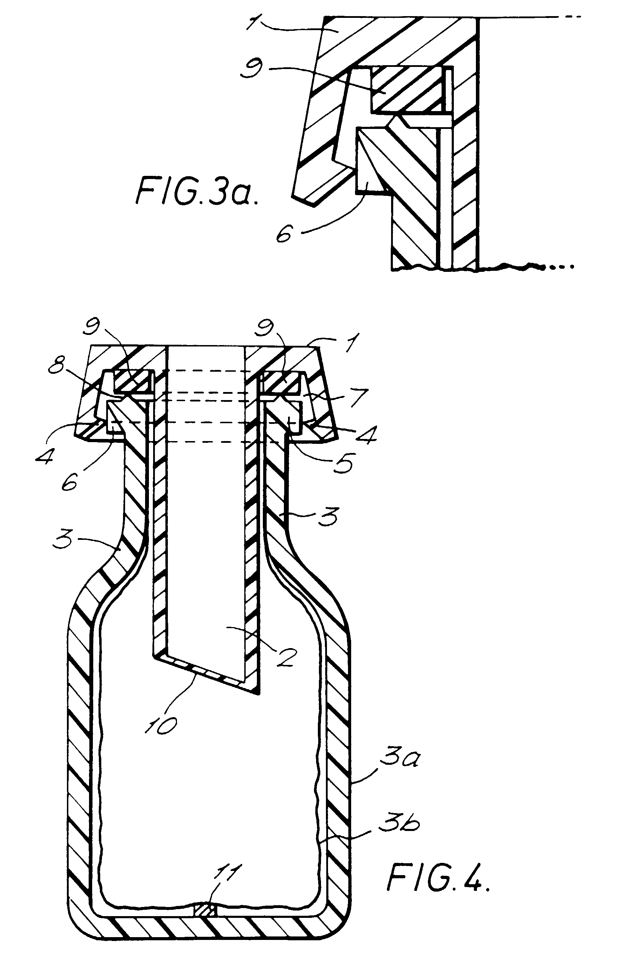

Fig. 2 zeigt den Behälterhals mit aufgesetzter Verschlußkappe (1) in Verschlußposition. Die Entlüftung erfolgt jedoch in diesem Fall in der Weise, daß Entlüftungsöffnungen (6) in Form von Aussparungen in dem umlaufenden Wulst (4) der Verschlußkappe (1) vorgesehen sind. Alternativ können auch Aussparungen in dem Ring (5) zur Entlüftung dienen (siehe Fig. 3). Der Eintauchstutzen (2) ist am unteren Ende mit einer Membran (10) abgeschlossen. Zur Entnahme von Flüssigkeit wird die Membrane mit einer Kanüle durchstoßen.

Figur 3 zeigt, ebenfalls in einem axialen Schnitt, einen Behälterhals mit aufgesetzter Verschlußkappe während des Verschließvorganges. Wie in Figur 3 dargestellt, befindet sich mindestens eine Entlüftungsöffnung (6) in dem Ring (5) des Behälters. Bevorzugt sind die Entlüftungsöffnungen in Form von Aussparungen in dem Ring (5) angeordnet. Alternativ können die Entlüftungsöffnungen in Form von Bohrungen angeordnet sein. Die Entlüftungsöffnungen (6) sind derart angeordnet, daß die Luft aus dem Kappeninnenraum (7) während des Verschließvorgangs durch die Entlüftungsöffnungen (6) entweichen kann, aber nach dem Verschließen des Behälters keine Verbindung mehr zwischen der Flüssigkeit im Inneren des Behälters und den Entlüftungsöffnungen besteht.

Fig. 4 zeigt in einem axialen Schnitt einen erfindungsgemäßen Behälter mit aufgesetzter Verschlußkappe, wobei die Entlüftungsöffnung(en) im äußeren Bereich des Rings (5) angeordnet ist (sind). Die Entlüftungsöffnungen können in Form von Aussparungen angebracht sein.

In einer bevorzugten Ausführungsform besteht der Behälter (3) aus einem formstabilen Außenbehälter und einem leicht verformbaren Innenbeutel (3b), der bei Flüssigkeitsentnahme in sich zusammenfällt. Bei diesen Ausführungsformen würden verbleibende Luftblasen sich besonders nachteilig auswirken. Solche Behälter sind beispielsweise in der Europäischen Patentschrift 532 873 beschrieben, auf die hiermit inhaltlich Bezug genommen wird. Die Vorrichtung (11) dient zur Befestigung des verformbaren Innenbeutels (3b) an der dem Beutel (3b) zugewandten Innenwand des äußeren starren Behälters (3a).

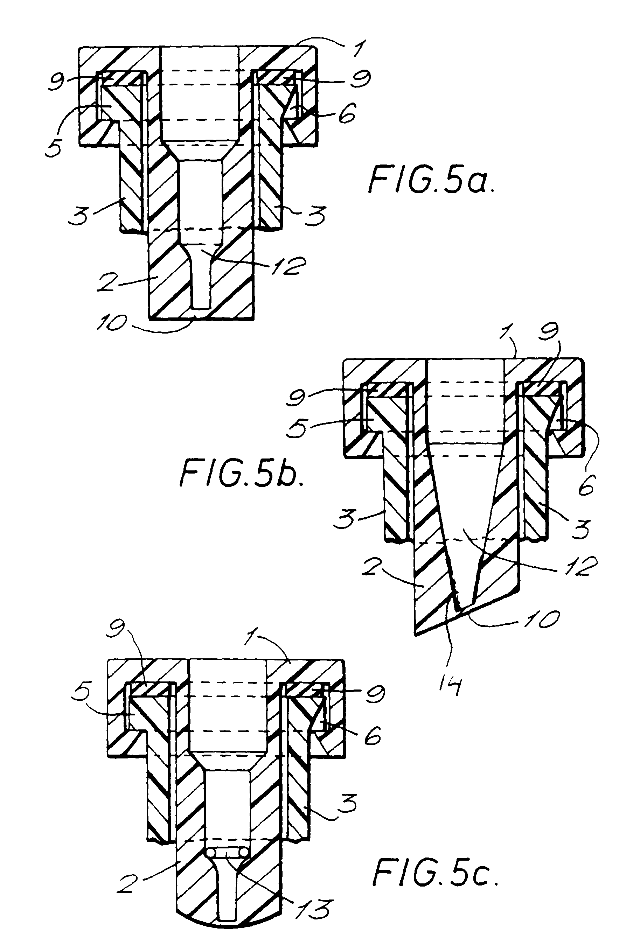

Die Figur 5a zeigt eine bevorzugte Ausführungsform der erfindungsgemäßen Verschlußkappe, bei der der Innenraum des Eintauchstutzens derart gestaltet ist, daß eine Führung (12) für eine Kanüle zur Flüssigkeitsentnahme ausgebildet ist. Im vorliegenden Fall sind die Entlüftungsöffnungen (6) am oberen Teil des Behälters (3) angebracht. Wie bereits beschrieben, können die Entlüftungsöffnungen alternativ auch an der Verschlußkappe angeordnet sein.

Die Figuren 5b, 5c zeigen andere Ausführungsformen hinsichtlich der Ausgestaltung des eintauchenden Stutzens (2) sowie der Führung (12) für die Kanüle zur Entnahme der Flüssigkeit.

Die Figur 5b zeigt eine Ausführungsform bei der die Führung (12) in eine Presspassung (14) übergeht. Der Preßsitz ist hinsichtlich Durchmesser und Länge so ausgelegt, daß einerseits der Widerstand zum Durchschieben der Knüle gering gehalten ist und andererseits eine ausreichende Dichtwirkung zwischen Stutzen und Kanüle erreicht wird.

Fig. 5c zeigt eine Ausführungsform mit einer elastischen O-Ring-Dichtung (13) zwischen Stutzen und einstehender Kanüle, wobei die Kanüle nicht abgebildet ist.

Wie in Fig. 5b und 5c gezeigt, kann das untere Ende des Eintauchstutzens mit der Membran (10) zweckmäßigerweise abgeschrägt sein, vorzugsweise um 40° bis 70° gegenüber der Stutzenachse. Dadurch wird ein Durchstoßen der Membran mit einer "stumpfen" Kanüle, deren Stirnfläche senkrecht zur Kanülenachse steht, erleichtert. Vorteile einer "stumpfen" gegenüber einer "spitz angeschliffenen" Kanüle liegen in der geringen Verletzungsgefahr für den Anwender, in dem geringeren Bearbeitungsaufwand zur Herstellung der Kanülen-Stirnfläche und der beim Einführen der Kanüle geringeren Gefahr des Partikelabriebs an der Stutzenwandung.

In den Zeichnungen ist ein Dichtungsring (9) sowie eine umlaufende Rippe (8) dargestellt. In einer alternativen Ausführungsform kann die umlaufende Rippe (8) weggelassen werden, wenn alle Teile paßgenau mit hoher Präzision hergestellt werden.

Für die Entnahme von Flüssigkeit aus dem Behälter (3) wird die Membran (10) mit einer Kanüle durchstochen. Bevorzugt sind Ausführungsformen, bei denen der Behälter (3) einen leicht verformbaren Innenbeutel (3b) aufweist und die Spitze der Kanüle sich in halber Höhe des Behälters befindet, wenn die Flüssigkeit entnommen wird. In diesem Fall wirken sich Luftblasen am wenigsten störend aus.

Behälter und Verschlußkappe (1) werden im allgemeinen aus Kunststoff gefertigt. Da die eingefüllte Flüssigkeit praktisch nicht kompressibel ist, muß das System aus Behälter und Verschlußkappe bei der Ausdehnung der Flüssigkeit in der Wärme ausreichend verformbar sein. Ebenso müssen bei der Entnahme der Flüssigkeit die Wandungen des Behälters ausreichend nachgeben bzw. zusammenfallen. Für die Herstellung solcher Behälter wie auch für die Verschlußkappe (1) stehen dem Fachmann geeignete Kunststoffe - beispielsweise aus Polyethylen oder Polypropylen - zur Verfügung.

## Patentansprüche

1. In Verschlußposition einrastende Verschlußkappe (1) für die gasblasenfreie Abfüllung von Flüssigkeiten, bei der ein nicht abdichtender Eintauchstutzen (2) vorgesehen ist, der während des Aufschiebens der Verschlußkappe (1) auf den Hals des Behälters (3) einen Teil des Behälterinhalts verdrängt, wobei eine oder mehrere Entlüftungsöffnung(en) (6) so an der Außenseite der Verschlußkappe (1) angeordnet ist (sind), daß diese während des Verschließens des Behälters bis zum Einrasten der innenliegenden am unteren Rand der Verschlußkappe umlaufenden Crimpkante (4) der Verschlußkappe (1) in die Verschlußposition mindestens eine Verbindung zwischen dem von der Verschlußkappe und Hals des Behälters gebildeten Verschlußkappeninnenraum (7) und der Außenseite herstellt (herstellen).

2. Verschlußkappe (1) nach Anspruch 1, bei der die Entlüftungsöffnung(en) (6) sich im oberen oder im seitlichen Teil der Verschlußkappe (1) befindet (befinden).

3. Verschlußkappe (1) nach Anspruch 1, bei der die Entlüftungsöffnung(en) (6) in Aussparungen in der Crimpkante (4) besteht (bestehen).

4. Verschlußkappe nach einem der Ansprüche 1 bis 3, dadurch gekennzeichnet, daß der untere Teil des Eintauchstutzens (2) der Verschlußkappe (1) abgeschrägt ist.

5. Verschlußkappe (1) nach Anspruch 1, dadurch gekennzeichnet, daß der Eintauchstutzen im unteren Teil mit einer leicht durchstechbaren Membran (10) versehen ist.

6. Verschlußkappe nach Anspruch 5, dadurch gekennzeichnet, daß der untere Teil des Eintauchstutzens abgeschrägt ist.

7. Verschlußkappe nach Anspruch 5 oder 6, dadurch gekennzeichnet, daß der innere Teil des Eintauchstutzens eine Führungseinrichtung für eine Kanüle aufweist.

8. Verschlußkappe nach einem der Ansprüche 5 bis 7, dadurch gekennzeichnet, daß die Führungseinrichtung (12) in eine Presspassung (14) übergeht.

9. Verschlußkappe nach einem der Ansprüche 5 bis 7, dadurch gekennzeichnet, daß der Stutzen mit einem 0-Ring-Dichtung versehen ist.

10. Behälter (3) mit in Verschlußposition einrastender Verschlußkappe (1) für die gasblasenfreie Abfüllung von Flüssigkeiten, bei der ein nicht abdichtender Eintauchstutzen (2) vorgesehen ist, der während des Aufschiebens der Verschlußkappe (1) auf den Hals des Behälters (3) einen Teil des Behälterinhalts verdrängt, wobei eine oder mehrere Entlüftungsöffnung(en) (6) so an der Außenseite der Verschlußkappe (1) angeordnet ist (sind), daß während des Verschließens des Behälters bis zum Einrasten der Verschlußkappe (1) in die Verschlußposition eine Verbindung nach außen hergestellt ist, die das Entweichen der aus dem Behälter verdrängten Gas- oder Flüssigkeitsmenge ermöglicht, wobei nach dem Verschließen des Behälters keine Verbindung mehr zwischen den Entlüftungsöffnungen (6) und der Flüssigkeit im Behälter (3) besteht.

11. Behälter nach Anspruch 10, dadurch gekennzeichnet, daß der Hals des Behälters (3) einen zylindrischen Ring (5) mit einer oder mehreren Entlüftungsöffnungen aufweist.

12. Behälter nach Anspruch 10 oder 11, dadurch gekennzeichnet, daß der Behälter unterhalb des starren Halses des Behälters aus einem leicht verformbaren Material (3b) besteht, so daß der Behälter bei einer Flüssigkeitsentnahme in sich zusammenfällt.

13. Behälter nach einem der Ansprüche 10, 11 oder 12, dadurch gekennzeichnet, daß der Behälter (3) aus einem starren äußeren Behälter (3a) und einem leicht verformbaren Innenbeutel (3b) besteht, der bei Flüssigkeitsentnahme in sich zusammenfällt.

14. Verfahren zur blasenfreien Abfüllung von Flüssigkeiten in einen Behälter (3), der einen seinen Hals umschließenden zylindrischen Ring (5) aufweist, dadurch gekennzeichnet, daß man zum Verschließen des Behälters eine in der Verschlußposition einrastende Verschlußkappe (1), die eine umlaufende Crimpkante (4) aufweist, auf den Hals des Behälters (3) aufschiebt, wobei an der Innenseite der Verschlußkappe (1) ein nicht-abdichtender Eintauchstutzen (2) ausgebildet ist, der einen Teil des Behälterinhalts verdrängt und während des Aufschiebens der Verschlußkappe (1) durch eine oder mehrere Entlüftungsöffnungen oder Aussparungen (6) an der Außenseite der Verschlußkappe (1), in dem zylindrischen Ring (5) oder in dem Wulst (4) eine Verbindung nach außen derart hergestellt ist, daß das Entweichen von Gas und/oder Flüssigkeit aus dem Behälter (3) ermöglicht wird, aber nach dem Verschließen des Behälters (3) mit der Verschlußkappe (1) keine Verbindung mehr zwischen der Flüssigkeit im Innern des Behälters und den Entlüftungsöffnungen (6) besteht.

## Claims

1. Closure cap (1) which engages in the closure position, for filling of liquids without gas bubbles, wherein a non-sealing dipping nozzle (2) is provided which displaces part of the contents of the container during the pushing of the closure cap (1) onto the neck of the container (3), one or more vents (6) being disposed on the outside of the closure cap (1) in such a way as to establish at least one communication between the closure cap interior space (7) formed by the closure cap and the neck of the container, and the outside, during the closing of the container until the crimped edge (4) of the closure cap (1) which runs round the inside of the lower edge of the closure cap engages in the closure position.

2. Closure cap (1) according to claim 1, in which the vent or vents (6) are located in the upper or side part of the closure cap (1).

3. Closure cap (1) according to claim 1, wherein the vent or vents (6) consists or consist of recesses in the crimped edge (4).

4. Closure cap (1) according to one of claims 1 to 3, characterised in that the lower part of the dipping nozzle (2) of the closure cap (1) is chamfered.

5. Closure cap (1) according to claim 1, characterised in that the dipping nozzle in the lower part is provided with a readily pierceable membrane (10).

6. Closure cap according to claim 5, characterised in that the lower part of the dipping nozzle is chamfered.

7. Closure cap according to claim 5 or 6, characterised in that the inner part of the dipping nozzle has guide means for a cannula.

8. Closure cap according to one of claims 5 to 7, characterised in that the guide means (12) merges into a press fit (14).

9. Closure cap according to one of claims 5 to 7, characterised in that the nozzle is fitted with an O-ring seal.

10. Container (3) having a closure cap (1) engaging in the closure position, for filling with liquids without gas bubbles, wherein a non-sealing dipping nozzle (2) is provided which displaces part of the contents of the container during the pushing of the closure cap (1) onto the neck of the container (3), one or more vents (6) being disposed on the outside of the closure cap (1) in such a way as to provide a connection with the outside during the closing of the container until the closure cap (1) has engaged in the closure position, this connection allowing the quantity of gas or liquid displaced from the container to escape, whilst after the container has been closed there is no further connection between the vents (6) and the liquid in the container (3).

11. Container according to claim 10, characterised in that the neck of the container (3) has a cylindrical ring (5) with one or more vents.

12. Container according to claim 10 or 11, characterised in that the container underneath the rigid neck of the container consists of a readily deformable material (3b), so that the container collapses in on itself when liquid is removed.

13. Container according to one of claims 10, 11 or 12, characterised in that the container (3) consists of a fixed external container (3a) and a readily deformable inner bag (3b) which collapses in on itself as liquid is removed.

14. Process for transferring liquids, without bubbles, into a container (3) which has a cylindrical ring (5) encircling its neck, characterised in that in order to close the container a closure cap (1), which engages in the closure position and has an encircling crimped edge (4), is pushed onto the neck of the container (3), a non-sealing dipping nozzle (2) being formed on the interior side of the closure cap (1), this dipping nozzle displacing some of the contents of the container, and, during the fitting on of the closure cap (1), a communication with the exterior is provided through one or more vents or recesses (6) on the outside of the closure cap (1), in the cylindrical ring (5) or in the bead (4), such that gas and/or liquid is able to escape from the container (3), but after the container (3) has been closed off with the closure cap (1) there is no further communication between the liquid inside the container and the vents (6).

## Revendications

1. Capuchon de fermeture (1) pour le remplissage de liquide sans formation de bulles de gaz, qui s'enclenche dans la position de fermeture et dans lequel est prévu un manchon plongeur (2) non étanche qui chasse une partie du contenu du récipient lors de l'enfilement du capuchon de fermeture (1) sur le goulot du récipient (3), une ou plusieurs ouvertures de mise à l'air (6) étant ménagées sur la paroi extérieure du capuchon de fermeture (1) de telle sorte que celles-ci établissent au moins une communication entre le volume intérieur (7), formé par le capuchon de fermeture et le goulot du récipient, et le côté extérieur pendant l'opération de fermeture du récipient jusqu'à l'enclenchement de l'arête périphérique (4) intérieure de sertissage, s'étendant sur le bord inférieur du capuchon de fermeture (1), jusque dans la position de fermeture.

2. Capuchon de fermeture (1) selon la revendication 1, dans lequel la ou les ouverture(s) de mise à l'air (6) se trouve(nt) dans la partie supérieure ou latérale du capuchon de fermeture (1).

3. Capuchon de fermeture (1) selon la revendication 1, dans lequel la ou les ouverture(s) de mise à l'air (6) est(sont) constituée(s) par des évidements dans l'arête de sertissage (4).

4. Capuchon de fermeture selon l'une quelconque des revendications 1 à 3, caractérisé en ce que la partie inférieure du manchon plongeur (2) du capuchon de fermeture (1) est chanfreinée.

5. Capuchon de fermeture (1) selon la revendication 1, caractérisé en ce que le manchon plongeur est pourvu, dans la partie inférieure, d'une membrane (10) facile à percer.

6. Capuchon de fermeture selon la revendication 5, caractérisé en ce que la partie inférieure du manchon plongeur est chanfreinée.

7. Capuchon de fermeture selon l'une ou l'autre des revendications 5 et 6, caractérisé en ce que la partie intérieure du manchon plongeur comprend un dispositif de guidage pour une canule.

8. Capuchon de fermeture selon l'une quelconque des revendications 5 à 7, caractérisé en ce que le dispositif de guidage (12) se transforme en un ajustage serré (14).

9. Capuchon de fermeture selon l'une quelconque des revendications 5 à 7, caractérisé en ce que le manchon est pourvu d'un joint torique.

10. Récipient (3) pour le remplissage de liquides sans formation de bulles de gaz, comportant un capuchon de fermeture (1) qui s'enclenche dans la position de fermeture et dans lequel est prévu un manchon plongeur (2) non étanche qui chasse une partie du contenu du récipient lors de l'enfilement du capuchon de fermeture (1) sur le goulot du récipient (3), une ou plusieurs ouvertures de mise à l'air (6) étant ménagées sur la paroi extérieure du capuchon de fermeture (1) de telle sorte qu'une communication vers l'extérieur est établie pendant l'opération de fermeture du récipient jusqu'à l'enclenchement du capuchon de fermeture (1) dans la position de fermeture, communication qui permet l'échappement de la quantité de gaz ou de liquide chassée hors du récipient, et après avoir refermé le récipient, il n'existe plus aucune communication entre les ouvertures de mise à l'air (6) et le liquide dans le récipient (3).

11. Récipient selon la revendication 10, caractérisé en ce que le goulot du récipient (3) comprend un anneau cylindrique (5) présentant une ou plusieurs ouverture(s) de mise à l'air.

12. Récipient selon l'une ou l'autre des revendications 10 et Il, caractérisé en ce que le récipient est constitué, au-dessous du goulot rigide du récipient, en un matériau facilement déformable (3b), de sorte que le récipient s'écrase lors d'un prélèvement de liquide.

13. Récipient selon l'une quelconque des revendications 10, 11, 12, caractérisé en ce que le récipient (3) est constitué par un récipient extérieur rigide (3a) et par un sac intérieur (3b) facilement déformable qui s'écrase lors d'un prélèvement de liquide.

14. Procédé pour le remplissage de liquides sans formation de bulles de gaz dans un récipient (3) qui comprend un anneau cylindrique (5) entourant son goulot, caractérisé en ce que pour refermer le récipient, on enfile sur le goulot du récipient (3) un capuchon de fermeture (1) qui s'enclenche dans la position de fermeture et qui comprend une arête de sertissage périphérique (4), dans lequel un manchon plongeur (2) non étanche est réalisé sur le côté intérieur du capuchon de fermeture (1) et chasse une partie du contenu du récipient, et pendant l'enfilement du capuchon de fermeture (1) une communication vers l'extérieur est établie par une ou par plusieurs ouverture(s) de mise à l'air ou évidements (6) sur le côté extérieur du capuchon de fermeture (1), dans l'anneau cylindrique (5) ou encore dans le bourrelet (4), de telle sorte que l'échappement de gaz et/ou de liquide hors du récipient (3) est possible, mais après avoir refermé le récipient (3) avec le capuchon de fermeture (1), il n'existe plus de communication entre le liquide dans l'intérieur du récipient et les ouvertures de mise à l'air (6).
